(19) Europäisches Patentamt
European Patent Office
Office européen des brevets

(11)  **EP 4 527 909 A1**

(12)  **EUROPEAN PATENT APPLICATION**
published in accordance with Art. 153(4) EPC

(43) Date of publication:
26.03.2025  Bulletin 2025/13

(21) Application number: 23806461.2

(22) Date of filing: 19.05.2023

(51) International Patent Classification (IPC):
$C10G\ 3/00^{(2006.01)}$      $C10G\ 11/18^{(2006.01)}$
$C10G\ 11/05^{(2006.01)}$     $C10L\ 1/02^{(2006.01)}$

(52) Cooperative Patent Classification (CPC):
C10G 3/00; C10G 11/05; C10G 11/18; C10L 1/02

(86) International application number:
PCT/BR2023/050153

(87) International publication number:
WO 2023/220798 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.05.2022  BR 102022009906

(71) Applicant: Petroleo Brasileiro S.A. - PETROBRAS
20031-912 Rio de Janeiro (BR)

(72) Inventors:
• RENE CHAMBERLAIN PRAVIA, Oscar
21941-915 Rio de Janeiro (BR)
• BELLO DE OLIVEIRA, Andre
21941-915 Rio de Janeiro (BR)
• DE REZENDE PINHO, Andrea
21941-915 Rio de Janeiro (BR)
• LOUREIRO XIMENES, Vitor
21941-915 Rio de Janeiro (BR)
• BRANDO BEZERRA DE ALMEIDA, Marlon
21941-915 Rio de Janeiro (BR)

(74) Representative: Clarke, Modet y Cía., S.L.
C/ Suero de Quiñones 34-36
28002 Madrid (ES)

(54)  **METHOD FOR GENERATING RENEWABLE PRODUCTS FROM BIO-OIL AND OIL STREAMS FROM CATALYTIC CRACKING**

(57)    The present invention belongs to the field of fluid catalytic cracking (FCC) processes for the production of fuels with fully renewable content, more specifically in the production of high octane gasoline (RON above 92 and/or MON above 83) and middle distillates. More specifically, the present invention relates to the co-processing of bio-oil and grease streams in the presence of an intermediate pore size zeolite catalyst, to produce more and better gasoline, reducing the amount of heavy fractions of low commercial value.

FIGURE 1

EP 4 527 909 A1

## Description

### Field of the invention

The present invention belongs to the field of fluid catalytic cracking (FCC) processes for the production of fuels with fully renewable content, more specifically in the production of high octane gasoline (RON ("Research Octane Number") above 92 and/or MON ("Motor Octane Number" above 83) and middle distillates. More specifically, the present invention relates to the co-processing of bio-oil and grease streams in the presence of a zeolite catalyst with intermediate pore size, to produce more and better gasoline, reducing the amount of heavy fractions of low commercial value and eliminating the use of flare oil to balance the heat balance of the unit.

Bio-oil is generated through fast pyrolysis, catalytic pyrolysis, hydrothermal liquefaction or other thermochemical processes for the conversion of lignocellulosic biomass.

### Description of the State of the Art

Brazil is one of the 195 signatory countries of the Paris Agreement, which aims to strengthen the global response to the threats arising from climate change caused by the emission of greenhouse gases. In this Agreement, each signatory country establishes its own commitments based on the so-called Nationally Determined Contributions (or NDC) and proposes what would be its contribution to the reduction of greenhouse gas (GHG) emissions, seeking to limit the global increase in average temperature to 2.0 °C above pre-industrial levels. The commitment made by the Brazilian NDC foresees a reduction in GHG emissions by 37% by 2025 compared to the 2005 value, and by 43% below 2005 levels by 2030. One of the routes to achieve this goal establishes the commitment to increase the share of sustainable bioenergy in its energy matrix to approximately 18% by 2030. With the launch of the RenovaBio program (Law # 13,576/2017 of December 26, 2017), the National Biofuels Policy was established, with the objective of promoting the adequate expansion of the production and use of biofuels in the national energy matrix and of offering predictability to the competitive participation of the several biofuels in the national fuel market.

Fluid catalytic cracking (FCC) is one of the main conversion processes, present in most oil refineries. It is mainly used for the production of high-octane gasoline through the use of zeolites that convert high molecular weight molecules into smaller molecules. Its technology is quite flexible, allowing the conversion of heavy petroleum streams, and its operating conditions can be optimized for the production of gasoline, propylene or products in the diesel oil range. FCC units can be found in most refineries in Brazil.

In the fluidized bed catalytic cracking process, hydrocarbon cracking reactions occur by the contact of a hydrocarbon stream with a catalyst in a dynamic flow regime, in a tubular reactor in ascending flow, also known as a riser, converting it into lighter hydrocarbon streams with greater economic value. Thus, hydrocarbon streams from petroleum refining with boiling points between 350 °C and 550 °C are converted into lighter hydrocarbons, predominantly gasoline constituents with a distillation range between 35 °C and 220 °C. Alternatively, liquid streams of renewable origin can also be used. The reaction temperatures (TRX) in the reactor are around 540 °C at the reactor outlet.

Cracking reactions are endothermic and the increase in the severity of the reaction makes it difficult to meet this energy demand. In conventional catalytic cracking, this thermal demand is met by burning the coke deposited on the catalyst in the reaction section. The catalyst is burned with air in a regeneration section at temperatures around 700 °C. In this way, its catalytic activity is reestablished, and the heated catalyst can be returned to the reaction section and provide the heat necessary for the endothermic reactions.

Modern regenerators operate in the dense phase of the regeneration bed, where most of the combustion of the coke deposited on the catalyst takes place, with minimum temperatures of around 680 °C and a catalyst residence time of around 4 minutes in the regenerator, which ensures the combustion of the coke. Typically, the maximum design temperature in the dense phase of the bed is 760 °C, while, operationally, this temperature is maintained at a maximum of 730 °C.

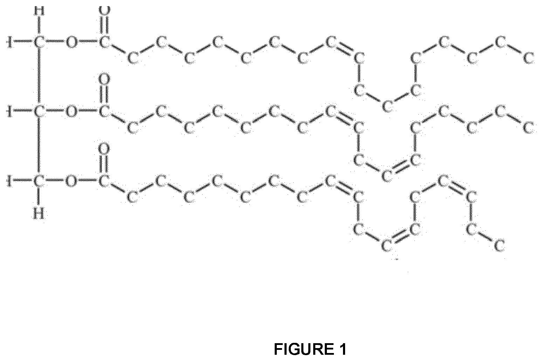

The problem of meeting thermal demand is aggravated if the feed streams used are grease (lipid) streams, such as soybean oil, castor oil and beef tallow. These streams of renewable origin have in their structure linear chains with up to 18 carbons, as shown in Figure 1, which shows a typical triglyceride structure. Linear carbon chains generally have 9 to 18 carbon atoms. These streams usually contain at least one unsaturation and have a low potential for coke formation in catalytic cracking.

Grease streams cannot be used directly as fuel but can be converted into higher value-added products such as aviation kerosene, diesel oil and BTX aromatics (benzene, toluene and xylenes).

Catalysts useful for catalytic cracking contain Y zeolites of the USY and REY types. In commercial catalytic cracking reactors, ZSM-5 zeolites (MFI), which have intermediate pore sizes, are also used to produce olefins, aromatics and increase the octane rating of gasoline. The term "intermediate pore-sized zeolite" is applied to any zeolite whose pores are intermediate in size to small-pore zeolites, such as type A zeolites, and large-pore zeolites, such as X and Y zeolites.

Intermediate zeolites are crystalline structures whose rings contain 10 or 12 oxygen atoms. These zeolites must have a silica-alumina ratio between 10 and 300. Examples of intermediate pore zeolites are, in addition to ZSM-5, the zeolites ZSM-8, ZSM-11 (also known as MEL), ZSM-12, ZSM-21, ZSM-23, ZSM-35, ZSM-38, IMF (also known as IM-5), TUN (also known as TNU-9) and EUO.

ZSM-5-based catalysts are essential for the cracking of oxygenated compounds of grease streams and the conversion of these compounds into hydrocarbons. The article "Catalytic cracking of mixtures of model bio-oil compounds and gasoil" indicates the existence of preferential interaction between ZSM-5 and oxygenated compounds, leading to greater removal of oxygen from the reaction products (GRAÇA, I; RAMÔA RIBEIRO, F.; CERQUEIRA, H.S.; LAM, Y.L.; DEALMEIDA, M.B.B.; Catalytic cracking of mixtures of model bio-oil compounds and gasoil. Applied Catalysis B: Environmental. 90(3-4): 556 to 563 (2009)).

However, the processing of grease streams in catalytic cracking leads to their thermal demand not being met for two main reasons: 1) the charge used itself is not a good precursor for coke formation, showing low Ramsbottom carbon residue; 2) ZSM-5 zeolites, as well as other zeolites with cavities smaller than zeolite Y, do not produce large coke deposition on the catalyst.

However, there is little literature related to solving the problem of energy deficit in catalytic cracking. Normally, this deficiency is solved by burning flare oil, also known as heating oil, in the regenerator. Under this approach, the regenerator becomes a combustor, where the burning of the oil generates enough heat to heat the catalyst. The regenerator bed must be heated by burning flare oil to temperatures around 700 °C. Dense phase temperatures (DPT) lower than 680 °C make it difficult to burn the heating oil in the regeneration bed and cause uncontrolled catalyst circulation towards the cracking reactor. The heat generated is transported to the reaction section through the catalyst itself. However, burning oil in the regeneration bed favors the occurrence of several problems in the operation of the regenerator. For example, the choice of heating oil to be used must be careful, since heating oils with a very low distillation point can cause afterburning, that is, combustion outside the bed. The temperature differential between the bed and the combustion gases can reach 300 °C, generating high temperatures in the cyclones and other equipment inside and outside the regenerator. Another possible problem is the wear of the dispersers responsible for introducing the heating oil into the regenerator. Early deactivation of the catalyst can also occur, due to the generation of high temperature points in the combustion bed. In addition, burning oil generates energy waste, which significantly increases the carbon footprint in catalytic cracking units. Additionally, flare oil can increase the presence of sulfur content in the fuels generated in the FCC.

Thus, the processing of grease streams in catalytic cracking units leads to an imbalance in the equilibrium of the unit, limiting the amount of renewable stream added to the converter. A solution to this technical problem is therefore necessary.

One way to mitigate the energy deficit created by the use of streams with low coke formation potential includes the use in catalytic cracking of catalysts containing zeolites modified with dehydrogenating metals such as nickel, which promote coke formation, as taught in the document WO 2010023456. Despite mitigating the energy deficit in cracking, the proposed solution was not able to eliminate it. Furthermore, the document does not propose the production of 100% renewable compounds, but rather the production of olefins.

The document US 7540952 teaches how to co-process grease streams with heavy vacuum diesel in catalytic cracking to maximize middle distillates. An example is the use of castor oil (castor oil or ricinus oil) as feed for an FCC pilot unit, using reaction temperatures between 300 °C and 400 °C, much lower than those used in a conventional FCC. Under these conditions, products in the diesel oil range are maximized, but with a small production of low-octane gasoline. The patent, however, does not teach how to deal with the growing energy deficit caused by the use of larger amounts of plant oil as feed for the FCC converter instead of vacuum diesel, which limits the use of this renewable fraction and, consequently, does not reveal the production of 100% renewable fuels with low sulfur contents.

The document BR 102014022366-5 also uses renewable grease streams to produce cracked naphtha in catalytic cracking reactors, using faujasitic catalysts. The naphtha produced has characteristics similar to those of cracked naphthas of fossil origin, with a high content of bioaromatics that have the advantage of having a low oxygenate content, giving these products a high calorific value when compared to alternative products such as ethers (ETBE and MTBE) and alcohols (ethanol and butanol). However, the document does not propose a solution to the energy deficit in catalytic cracking caused by the use of the grease stream, nor the production of 100% renewable fuels. Furthermore, the document BR 102014022366-5 also points out the need for an oxytreatment step of the naphtha formed, in order to adjust the sulfur content to less than 10 ppm and the benzene content to less than 1%.

However, some renewable streams, even if liquid, have chemical characteristics that are very different from those of grease streams, and have a high potential for coke formation. Among these, lignocellulosic streams can be mentioned, such as pyrolysis oil, or bio-oil, resulting from the rapid pyrolysis process of biomass. Bio-oil consists of a mixture of several families of oxygenated compounds such as aldehydes, ketones, and phenolic compounds. It has a high aromatic content and a high potential for coke formation in catalytic cracking and should not be confused with lipid streams. The document BR 102016003995-9 discloses a process for obtaining biofuels, particularly high-octane gasoline, through the co-processing of a feedstock comprising pyrolysis oil (bio-oil), a gaseous stream of light saturated hydrocarbons, rich in hydrogen, and a main fossil feedstock, in the FCC process. It uses two reaction sections in its process: reaction temperature in the range of 500 °C to 800 °C in the first reaction section and in the range of 500 °C to 620 °C in the second reaction section. Bio-oil, a plant stream described in the document, does not generate an energy deficit in catalytic cracking, as it is of lignocellulosic origin, showing a high potential for coke formation. Thus, unlike the present invention, the bio-oil stream must be introduced separately into the riser reactor, as it is not miscible with the main (fossil) feedstock. Additionally, the document is not aimed at the production of 100% renewable compounds. In this specific case, the bio-oil is introduced before the diesel input to minimize the coke yield. When the bio-oil is introduced after the reference diesel input, the coke yield is maximized.

The document BR 1120170232529 combines hydropyrolysis with hydroprocessing steps for the production of fuels from lignocellulosic biomass. However, the aforementioned document does not propose the use of catalytic cracking of bio-oil. Thus, the invention described in the patent BR 1120170232529 deals with the direct hydrogenation of bio-oil obtained by hydropyrolysis using the gas stream generated in the process. This approach shows economic disadvantages related to the quality of the bio-oil (high water content, acidity and carbon residue) that require high severity, with impact on reactor engineering, in addition to high hydrogen consumption for the process. Therefore, the present invention brings advances by being able to insert the bio-oil into the catalytic cracking without the inconveniences brought by the hydrogenation process.

Thus, there is no solution in the state of the art that allows the generation of totally renewable products in catalytic cracking with thermal equilibrium. The solutions suggested for the production of renewable compounds in the state of the art lead the converter to a thermal deficit as the percentage of grease streams in the feed increases. In order to solve the problems mentioned, the present invention was developed, which proposes introducing a fraction of a bio-oil generated through a thermochemical biomass conversion technology, such as fast or slow pyrolysis, into the catalytic cracking of a grease stream, for the production of 100% renewable fuels with a sulfur content of less than 10 ppm.

Thus, the aforementioned process allows the production of 100% renewable fuels with a sulfur content of less than 10 ppm and with a balance in the thermal balance of the FCC unit, eliminating the use of flare oil, thus maintaining the economy and energy efficiency of the process.

In this context, the present invention proposes the production of 100% renewable fuels from abundant raw materials such as lignocellulosic biomass, such as sugarcane straw and bagasse.

The present invention is applicable to catalytic cracking units in which full sustainability of the FCC can be achieved, using grease streams together with a stream fraction originating from the thermochemical conversion of lignocellulosic material, such as a fraction of pyrolysis bio-oil (fast or slow), catalytic pyrolysis or hydrothermal liquefaction, thus increasing the market gain potential of this route, due to the great availability of lignocellulosic raw materials.

## Brief Description of the Invention

As a preliminary, it is emphasized that the description that follows will be based on preferred embodiments of the invention. As will be evident to any person skilled in the art, however, the invention is not limited to these particular embodiments, but only to the scope of protection defined in the claims.

The present invention relates to a catalytic cracking process for the production of fully renewable fuels that use grease streams and a bio-oil fraction as feedstock, characterized by the co-processing of these renewable streams in fluid catalytic cracking in contact with a zeolite catalyst with intermediate pore size.

The present invention generates products in the range of high octane MON and RON gasoline, suitable for Otto cycle engines. The products generated by the present invention in the other distillation ranges provide fuels with lower carbon emissions into the atmosphere and a sulfur content of less than 10 ppm, that is, with desirable characteristics for bunker (marine fuel oil), fuels for Otto cycle engines and fuels for Diesel cycle engines.

The objective of the present invention is also to develop a process capable of transforming streams of renewable origin into a product that can be incorporated into the fuel pool, but with low carbon emissions.

The invention can be applied to any existing catalytic cracking unit, provided that small modifications are made to the feed system of the unit, ensuring the segregated injection of bio-oil into the unit, or even its mixing with the grease stream immediately before entering the reactor.

The present invention provides a solution to the energy deficit caused by the use of grease streams in catalytic cracking by means of the use of bio-oil, whose high coke generation potential rebalances the thermal balance of catalytic cracking, eliminating the use of flare oil.

## Description of the Drawings

The present invention will be described in more detail below, with reference to the attached figures which, in a schematic manner and not limiting the inventive scope, represent examples of its implementation. The drawings include:

- Figure 1 illustrating the chemical structure of a typical grease stream;
- Figure 2 illustrating in a simplified manner the block diagram of the process of the present invention.

**Detailed Description of the Invention**

The present invention relates to a process for producing fully renewable fuels, in particular gasoline and middle distillate streams, by fluid catalytic cracking of a load comprising bio-oil and grease streams of renewable origin.

The term "bio-oil" used herein is defined as "the product obtained from the thermochemical conversion of a lignocellulosic biomass with a lignin content greater than 20% by mass"

Among the plant biomasses useful for the production of bio-oil, forestry residues such as those from the paper and cellulose industry, agricultural residues such as sugarcane bagasse and sugarcane straw can be mentioned.

The grease stream used as load can be selected from plant oil streams and animal fats.

According to the present invention, the feeding of grease streams into catalytic cracking units, a feed with low coke generation potential, is compensated by the use of bio-oil, a feed with high coke generation potential. This combination allows the thermal balance to be balanced during cracking, eliminating the need for flare oil. The air preheating furnace, which heats the air fed into the regeneration section, would be an additional alternative to the use of flare oil. It is generally used in cracking units only during unit startup after maintenance shutdowns. However, like flare oil, it increases the carbon footprint of the process, as it uses a fossil current in the furnace to generate heat, taking the temperature from around 220 °C at the compressor outlet to temperatures of around 500 °C at the regeneration bed inlet.

The present invention also allows the production of gasoline containing a high content of aromatic compounds, such as benzene, toluene, xylene, alkylated phenols and furans.

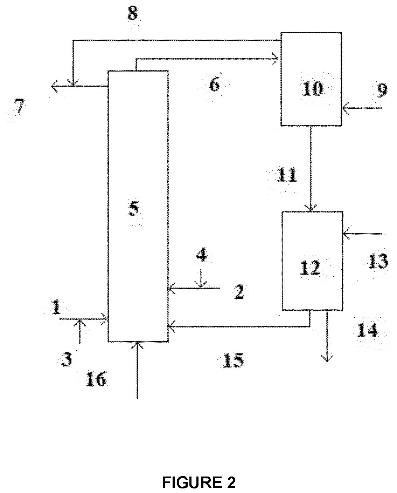

The invention is described in detail below with the aid of Figure 2, which presents, in a simplified manner, the block diagram of the process in a fluid catalytic cracking unit for the processing of grease and bio-oil streams, aimed at the production of fuels with the characteristic of being 100% renewable.

A grease stream (1) and bio-oil (2) are introduced in this order into a fluid catalytic cracking reactor (5), using water vapor (3) and (4) as auxiliary fluid for atomization, thus coming into contact with a zeolite catalyst with intermediate pores (15), coming from the regenerator (12). The catalyst is raised towards the reaction zone by means of lift steam (16). The feed and the catalyst come into contact under the following conditions: contact time in the range of 1.0 to 3.0 seconds; catalyst-to-oil ratio between 5 and 30, pressures in the range of 200 kPa to 400 kPa, reaction temperature between 400 °C and 520 °C.

At the reactor outlet, at the end of the reactions, the coke-deactivated catalyst (6) is separated from the products of the cracking reactions.

The coke-deactivated catalyst (6) goes to a rectification stage (10) where it receives an inert gaseous stream (9), preferably water vapor, to remove light hydrocarbons (8) that are directed to be mixed with the products already separated, composing the hydrocarbon stream (7) obtained in the process.

After rectification (10), a deactivated catalyst (11) proceeds to a regeneration stage (12) by combusting the coke in the presence of air (13), resulting in combustion gases (14) whose main components are carbon monoxide, carbon dioxide, nitrogen and unreacted oxygen.

The regenerated catalyst (15) returns to the reactor (5) at a temperature high enough to provide heat for the endothermic reactions of the process, thus completing a cycle of the process of the present invention.

The hydrocarbon streams obtained (7) in the process comprise: fuel gas (hydrogen, C1 and C2) which includes ethene, light (C3 and C4); naphtha (C5+, 220 °C); medium (220 °C, 344 °C) and bottom products (> 344 °C).

The grease stream of the feed (1) of the present invention may be a biomass of plant or animal origin composed of mono, di, or triglycerides, in which the oil of renewable origin may be castor oil, soybean oil, cottonseed oil, beef tallow or any other triglyceride or reaction product of its transesterification with methanol or ethanol.

In an alternative embodiment of the present invention, the grease stream (1) is mixed with the bio-oil (2) in a mixer not shown in Figure 2.

The zeolite catalyst with intermediate pore size contains a crystalline structure selected from: MFI, MEL, ZSM-8, ZSM-12, ZSM-21, ZSM-23, ZSM-35, ZSM-38, IMF and TUN and any of these combinations.

The typical catalyst comprises 10% to 75% w/w of zeolite with intermediate pore size, 1% to 40% w/w of alumina, 0% to 20% w/w of silica, 0% to 20% w/w of phosphorus; and kaolin for closing the balance.

The fluid catalytic cracking reactor is of the riser type.

The gasoline produced can be subsequently hydrogenated at temperatures between 350 °C and 390 °C, using as catalyst a sulfide system based on $CoMo/Al_2O_3$, containing from 2.5% to 6% by mass of cobalt and approximately 7 to 10% by mass of molybdenum.

The present invention also allows the production of middle distillates intended for the production of renewable diesel or use as bunker.

Thus, in a first embodiment, the present invention deals with a process for generating a wide range of renewable products, with quality suitable for incorporation into the renewable diesel pool, or use as renewable bunker, or even incorporation into the renewable gasoline pool, which comprises the steps: (a) mixing grease stream with bio-oil, and (b) converting the mixture in the catalytic cracking process.

The present invention also describes a process for obtaining renewable fuels from the combined use of two distinct renewable loads: bio-oil and the grease stream. It should be noted that the bio-oil comes from lignocellulosic biomass conversion processes such as pyrolysis or hydrothermal liquefaction or even mixtures of bio-oils obtained by these routes. The biomasses used for the production of bio-oil can be, among others, sugarcane straw and bagasse.

In a second embodiment, the present invention considers a formulation of renewable diesel for Diesel cycle engines, renewable bunker, renewable gasoline for Otto cycle engines, which comprises the conversion in catalytic cracking of a stream containing grease compounds and bio-oil, according to the process described above.

The invention will be better understood through the brief description of its examples.

EXAMPLES:

For this study, the following tests were performed, which represent examples of the present invention, where the results obtained by the state of the art and the present invention in catalytic cracking are shown.

A set of experiments were performed in ranges of interest of the process variables so that the influences of the main variables on the results achieved were known.

**EXAMPLE 1:** Yields of processing heavy vacuum gas oil and co-processing of bio-oil with heavy vacuum gas oil.

1.1 - Feed streams:

In the experiments, bio-oil produced from pine wood was co-processed, whose properties are shown in Table I, together with heavy vacuum gas oil. Another test, in which pure heavy vacuum gas oil was processed, was used as a reference, as shown in Table II.

**Table I**

| Characterization of bio-oil | | |
|---|---|---|
| **Analysis** | **Method** | **Wood** |
| **Density 20/4 °C** | ASTM D4052 | 1,2113 |
| **Carbon residue, wt%** | ASTM D4530 | 20.9 |
| **Sulphur, mg/kg** | Petrobras | < 33 |
| **Basic nitrogen, mg/kg** | UP 269-90 | 206 |
| **Total acidity, mg KOH/g** | ASTM D664 | - |
| **Carbon (C), wt%** | Petrobras | 43.35 |
| **Hydrogen (H), wt%** | Petrobras | 7.93 |
| **Oxygen (O), wt%** | ASTM D5622 | 49.14 |
| **Ash, wt%** | ASTM D482 | 0.078 |
| **Water, wt%** | ASTM D95 | 38.13 |
| **Viscosity at 40 °C, mm$^2$/s** | ASTM D445 | 62.026 |
| **Viscosity at 60 °C, mm$^2$/s** | ASTM D445 | 19.532 |
| **Viscosity at 80 °C, mm$^2$/s** | ASTM D445 | 8.1812 |
| **Metals, mg/kg** | ASTM D5708 | |
| **Fe** | ASTM D5708 | 8.74 |
| **Ni** | ASTM D5708 | 0.09 |
| **Na** | ASTM D5708 | 2.15 |
| **V** | ASTM D5708 | 0.01 |
| **Flash point, °C** | ASTM D93 | 54 |
| **Pour point, °C** | ASTM D97 | < -12 |

(continued)

| Characterization of bio-oil | | |
|---|---|---|
| **Analysis** | **Method** | **Wood** |
| **Lower calorific value, MJ/kg** | ASTM D240 | 16.02 |
| **Higher calorific value, MJ/kg** | ASTM D240 | 17.70 |

**Table II**

| Characterization of heavy vacuum diesel | | |
|---|---|---|
| | | **Heavy vacuum diesel** |
| **Density 20/4** | | 0.9409 |
| **API** | | 18.3 |
| **Sulphur, mg/kg** | ASTM D 5453 | 0.65 |
| **Total nitrogen, mg/kg** | ASTM D 5762 | 0.26 |
| **Basic nitrogen, mg/kg** | N-2373 | 1312 |
| **Aniline point, °C** | NBR-11343 | 84.9 |
| **RCR, wt%** | ASTM D 4530 | 1.92 |

1.2 - Analysis:

The following yield groups were defined: fuel gas (methane, hydrogen, ethane and ethylene), LPG (C3 and C4 hydrocarbons, except propylene), propylene, naphtha (C5-220 °C), LCO (220-343 °C), bottoms (+ 343 °C), coke, carbon monoxide, carbon dioxide and water.

The coke yield was calculated from the mass flow rate of the combustion gas and its chromatographic composition.

In general, studies in the literature calculate the water produced by the difference between 100% by weight and the sum of the other yields or simply do not report how the water yield was calculated. In this example, the water yield was estimated by totaling the water calculated at the end of each experiment subtracted from the amount fed to the unit (lifting, dispersion, rectification and separation devices).

1.2 - Test unit:

A demonstration-scale FCC unit (U-144) operating at a load flow rate of 120 kg/h was used. The catalyst inventory of the unit is extremely high, around 300 kg. The unit has an adiabatic temperature control system for the main equipment: riser reactor, rectifier, and regenerator, which allows studies involving the energy aspects of the process to be carried out.

The bio-oil was injected after the introduction of the heavy vacuum gas oil, the most appropriate position for maximizing the coke yield in the unit, as taught in the document BR 102016003995-9.

From the test yields with the reference diesel (A1) and the co-processing of 20% bio-oil (BO) and 80% diesel (A2), the presumed yield of pure bio-oil (A3) was calculated using equations 1 and 2:

$$\text{Pure BO yield } i = \left( \text{Co-processing yield } i - \text{GOP yield } i \left( \% \frac{\text{Co-processing GOP}}{100} \right) \right) / \left( \% \frac{\text{Co-processing BO}}{100} \right)$$

Equation 1

Since the percentages of diesel and bio-oil were 80% and 20%, respectively, then:

**Pure BO yield i = (Co-processing yield i – GOP yield i (0.8))/(0.2)**

Equation 2

The octane values MON and RON obtained in the co-processing of bio-oil and diesel (A2) are higher than those obtained with diesel only (A1). It was conservatively assumed that the octane values of A3 are the same as those of A2.

**Table III**

| Operating conditions in U-144 and efficiency (% by weight in relation to the fuel load) | | | |
|---|---|---|---|
| Test | A1 | A2 | A3 |
| Diesel (wt%) | 100 | 80 | 0 |
| Bio-oil (wt%) | 0 | 20 | 100 |
| Yields | | | |
| Fuel gas (hydrogen, C1-C2) (wt%) | 4.0 | 3.6 | 1.8 |
| GLP (C3-C4) (wt%) | 16.2 | 13.8 | 4.1 |
| Gasoline (PIE-220 °C) (wt%) | 42.9 | 36.0 | 8.6 |
| LCO (220 °C to 344 °C) (wt%) | 17.0 | 14.2 | 2.7 |
| Bottoms (+ 344 °C) (wt%) | 12.3 | 7.8 | -10.3 |
| Coke (wt%) | 7.4 | 9.8 | 19.2 |
| Carbon monoxide (wt%) | 0.05 | 1.3 | 6.1 |
| Carbon dioxide (wt%) | 0.09 | 0.6 | 2.8 |
| Water (wt%) | 0.0 | 13.0 | 65.0 |
| Total (wt%) | 100.0 | 100.0 | 100.0 |
| Quality of gasoline | | | |
| MON | 81.6 | 83.9 | 83.9 |
| RON | 91.2 | 92.7 | 92.7 |

**EXAMPLE 2:** Yields from biodiesel processing and co-processing of biodiesel and bio-oil.

2.1 - Feed streams:

In the prior art experiments, a biodiesel (fatty acid methyl ester) was used as a model molecule containing mainly fatty chains with approximately 18 carbon atoms, the properties of which are shown in Table IV. The bio-oil used in the experiments of the present invention was produced from pine wood and its properties are shown in Table I.

**Table IV**

| Characterization of biodiesel (grease source stream) | | |
|---|---|---|
| Analysis | Method | Result |
| Flash point (°C) | ASTM D93 | 150.0 |
| Density 20/4 °C | ASTM D4052 | 0.8800 |
| Aspect/color | Visual | Brown |
| Acidity index (mg KOH/g) | EN ISO 14104 | 0.35 |
| Cold plugging point (°C) | NBR 14747 | 1 |
| Oxidation stability 110 °C (h) | EN 14112 | 9.0 |
| Water content KF (mg/kg) | EN ISO 12937 | 340 |
| Viscosity at 40 °C (cSt) | ASTM D445 | 4.2559 |
| Viscosity at 60 °C (cSt) | ASTM D445 | 2.9466 |
| Ash content (wt%) | ASTM D482 | 0.001 |

(continued)

| Characterization of biodiesel (grease source stream) | | |
|---|---|---|
| Analysis | Method | Result |
| Asphaltenes with n-heptane (wt%) | ASTM D6560 | < 0.50 |
| Basic nitrogen (mg/kg) | UP 269-10 | 1.9 |
| Higher calorific value (cal/kg) | ASTM D240 | 9557 |
| Micro carbon residue (wt%) | ASTM D4530 | < 0.01 |
| Aniline point (°C) | NBR 11343 | < 0 |
| Sulphur (mg/kg) | ASTM D5453 | < 5 |
| Total nitrogen (mg/kg) | ASTM D5453 | 17.30 |
| Fe (mg/kg) | ASTM D5708 | 0.19 |
| Ni/V (mg/kg) | ASTM D5708 | < 0.01/< 0.01 |
| Na/Zn (mg/kg) | ASTM D5708 | 0.08/0.01 |

2.2 - Test unit:

The same demonstration-scale FCC unit described in Example 1 was used. The total feed flow rate was maintained at 130 kg/h. The grease stream was fed into the unit at 150 °C. The riser pressure was maintained at 257 kPa and the reaction temperature at 500 °C. To maintain the heat balance, the air fed into the regenerator was heated to 500 °C.

2.3 - Analyses:

The following output groups were defined: fuel gas (methane, hydrogen, ethane and ethylene), LPG (C3 and C4 hydrocarbons, except propylene), propylene, naphtha (C5-220 °C), LCO (220-343 °C), bottoms (+ 343 °C), coke, carbon monoxide, carbon dioxide and water.

The coke yield was calculated from the mass flow rate of the combustion gas and its chromatographic composition. Samples of the total liquid effluent were collected to perform simulated distillation (ASTM D2887).

In general, studies in the literature calculate the water produced by the difference between 100% by weight and the sum of the other yields or simply do not report how the water yield was calculated. In this example, the water yield was estimated by totaling the water calculated at the end of each experiment subtracted from the amount fed to the unit (lifting, dispersion, rectification and separation devices).

2.4 - Experimental results:

The results in Table V show the product yields and the operating conditions used in the tests.

In the test related to the state of the art (B1), only biodiesel was used as feed for catalytic cracking. In B1, there was a need for continuous use of flare oil to maintain the dense phase temperature at 600 °C, sufficient to provide combustion of the coke deposited on the catalyst at the appropriate speed.

In the tests related to this invention (B2 to B4), the bio-oil (BO) is co-processed with the grease stream (BD) to ensure the thermal balance of the unit. The yields of experiments B2, B3 and B4 were calculated from the yields of A3 (Table III) and B1 (Table V) by Equation 3.

$$\textbf{Co-processing yield i} = \%\frac{BO}{100} \cdot \textbf{A3 yield i} + \%\frac{BD}{100} \cdot \textbf{B1 yield i}$$

Equation 3

where BO is the percentage of bio-oil in the FCC feed and BD is the percentage of biodiesel, the grease stream, in the FCC feed.

To calculate the flare oil flow rates for experiments B2 and B3, as well as the regeneration temperature for B4, it was considered that the heat of combustion of coke and flare oil are 8740 kcal/kg and 10100 kcal/kg, respectively.

In test B2, 5% bio-oil is co-processed with 95% biodiesel. The flow rate of flare oil required to maintain the regenerator temperature at 600 °C was calculated, in a simplified way, from the combustion heats of coke and flare oil in test B1 and from Equation 4.

$$\text{Heat B1 (kcal/h)} = 10100. \text{ flow rate of flare oil B1} + 8740. \text{ \% Yield Coke B1./100.}$$

$$\text{total flow rate B1}$$

$$\text{Heat B1} = 37149 \text{ kcal/h}$$

$$\text{Flow rate of flare oil (kg/h)} = (37149 - 8740. \text{ \% Yield Coke/100. total flow}$$

$$\text{rate)/10100}$$

Equation 4

In test B2, it is still necessary to use a flow rate equivalent to 1.1 kg/h of flare oil for combustion in the regenerator and maintenance of the thermal balance. The bio-oil injected downstream of the grease stream allows the coke yield to be maximized to meet the thermal balance as taught by the document BR 102016003995-9.

The co-processing of 9% bio-oil and 91% biodiesel (test B3) makes it possible to dispense with flare oil to maintain the thermal balance of the unit in accordance with Equation 4.

Test B4 shows the results of 12% bio-oil and 88% biodiesel. In this case, there was an increase in the dense phase temperature of the regeneration from 600 °C to 611 °C.

High conversions were achieved in all cases, greater than 85 wt%.

The MON and RON octane values for the B1 test are extremely high, 96.8 and 102.1, respectively.

**Table V**

| U-144 operating conditions, yields (wt% relative to the feedstock), aromatic and phenolic concentrations | | | | |
|---|---|---|---|---|
| **Test** | **B1** | **B2** | **B3** | **B4** |
| **Grease stream (wt%)** | 100 | 95 | 91 | 85 |
| **Bio-oil (wt%)** | 0 | 5 | 9 | 12 |
| **Yields** | | | | |
| **Fuel gas (wt%)** | 4.3 | 4.2 | 4.1 | 4.0 |
| **GLP (C3-C4) (wt%)** | 26.8 | 25.7 | 24.8 | 24.1 |
| **Naphta (C5-220 °C) (wt%)** | 42.3 | 40.6 | 39.3 | 38.3 |
| **LCO (220 °C to 344 °C) (wt%)** | 9.3 | 9.0 | 8.7 | 8.5 |
| **Bottoms (+ 344 °C) (wt%)** | 3.3 | 2.6 | 2.1 | 1.7 |
| **Coke (wt%)** | 1.4 | 2.3 | 3.0 | 3.5 |
| **Water (wt%)** | 8.5 | 11.3 | 13.5 | 15.2 |
| **CO (wt%)** | 3.6 | 3.7 | 3.8 | 3.9 |
| **$CO_2$ (wt%)** | 0.5 | 0.6 | 0.7 | 0.8 |
| **Total (wt%)** | 100.0 | 100.0 | 100.0 | 100.0 |
| **Regenerator conditions** | | | | |
| **Regenerator temperature (°C)** | 600 | 600 | 600 | 611 |
| **Flare oil flow rate (kg/h) [a]** | 2.1 | 1.1 | 0 | 0 |
| **Quality of gasoline** | | | | |
| **MON** | 96.8 | - | - | - |
| **RON** | 102.1 | - | - | - |
| a - Flare oil flow rate calculated with the coke yield estimated by equation 3, applied to equation 4. | | | | |

**EXAMPLE 3:** Yields of soybean oil processing and co-processing of soybean oil and bio-oil in an ACE benchtop unit.

3.1 - Loads:

In the prior art experiments, a soybean oil containing mostly fatty chains with approximately 18 carbon atoms was used, whose properties are shown in Table VI. The bio-oil used in the experiments of the present invention was produced from pine wood and its properties are similar to those shown in Table VII. The bio-oil was mixed with soybean oil at levels of 1, 5, 10 and 20% by weight. In all mixtures, 1% by weight of the commercial dispersant TWEEN 80 from Sigma Aldrich was used.

**Table VII**

| Characterization of bio-oil | | |
|---|---|---|
| **Analysis** | **Method** | **Result** |
| **Specific gravity by digital densitometer at 20 °C, g/cm$^3$** | Petrobras | 1.181 |
| **Micro carbon residue, wt%** | Petrobras | 13.2 |
| **Total sulfur by UV fluorescence, mg/kg** | Petrobras | 89.3 |
| **Nitrogen, mg/kg** | ASTM D4629 | 533 |
| **Total acidity, mg KOH/g** | ASTM D664 | - |
| **Carbon (C), wt%** | Petrobras | 35.4 |
| **Hydrogen (H), wt%** | Petrobras | 6.9 |
| **Oxygen (O), wt% (by difference)** | Petrobras | 57.7 |
| **Water, wt%** | ASTM D95 | 36.0 |
| **Viscosity at 40 °C, mm$^2$/s** | ASTM D445 | 8.59 |
| **Viscosity at 60 °C, mm$^2$/s** | ASTM D445 | 4.135 |

**Table VII**

| Characterization of soybean oil (fatty source stream) | | |
|---|---|---|
| **Analysis** | **Unity** | **Value** |
| **Density 20/4 °C** | | 0.9194 |
| **Iodine index** | g Iodine/100 g | 145.2 |
| **Viscosity at 30 °C** <br> **Viscosity at 40 °C** <br> **Viscosity at 50 °C** | mm$^2$/s | 46.34 <br> 32.76 <br> 23.97 |
| **Carbon (C)** | wt% | 76.8 |
| **Hydrogen (H)** | wt% | 11.4 |
| **Sulphur (S)** | wt% | < 0.3 |
| **Oxygen (O)** | wt% | 11.4 |
| **Total nitrogen (Antek)** | mg/kg | 2.93 |
| **Chemical elements in diesel, biodiesel and plant oils** <br> **K** <br> **Na** <br> **Ca** <br> **Mg** <br> **P** <br> **Si** | mg/kg | < 1.0 <br> 2.3 <br> < 1.0 <br> < 1.0 <br> < 1.0 <br> - |

(continued)

| Characterization of soybean oil (fatty source stream) | | |
|---|---|---|
| **Analysis** | **Unity** | **Value** |
| **Smoke point*** | °C | Min. 210 |
| **Acidity index*** | mg KOH/g | Max. 0.20 |
| **Humidity and volatiles*** | wt% | Max. 0.10% |
| **Impurities*** | wt% | Max. 0.05% |
| * Specification | | |

3.2 - Test unit:

An ACE bench catalytic test was used. The catalyst mass used was 9 g for each test. The total feed flow rate was maintained at 1.2 g/min for an injection time of 64 s, which is equivalent to a catalyst-to-oil ratio of 7.0. The grease stream is fed into the unit at 50 °C. The reactor is controlled at a temperature of 500 °C.

After the injection of the load, the reactor bed continues to be fluidized by the nitrogen flow to remove any product that is absorbed between the catalyst particles or on their surface. This step is called catalyst rectification and lasts approximately 350 s.

After this, part of the nitrogen flow is replaced by synthetic air so that the coke on the catalyst surface is converted to carbon dioxide. To ensure that the complete combustion process occurs, the reactor outlet flow passes through a catalytic converter that oxidizes all the monoxide to carbon dioxide. The water formed is retained by a desiccator and the dioxide is quantified in an infrared analyzer. The coke yield is calculated from this value. This coke burning stage is called regeneration, since the catalyst has its activity partially restored due to the removal of the coke from its surface.

The liquid product corresponds to the high molecular weight hydrocarbon fraction, characterized by compounds with a carbon number greater than 6 (six). In the processing of renewable loads such as soybean oil and bio-oil, oxygenated compounds and water may also appear. The compounds that leave the reactor in the gas phase are condensed and stored in chromatography vials, adapted to condensers immersed in a bath at a temperature of approximately 15 °C, to prevent the products formed from volatilizing. This bath is a 1:1 mixture of water and ethylene glycol. The condenser containing the liquid is weighed and quantified by gas chromatography using an analysis called simulated distillation, where the products are grouped according to their boiling point, corresponding to certain hydrocarbon ranges.

3.3 - Analysis:

The following yield groups were defined: fuel gas (methane, hydrogen, ethane and ethylene), LPG (C3 and C4 hydrocarbons), propylene, liquid products (naphtha, LCO, bottoms and water), naphtha (C5-220 °C), LCO (220 to 343 °C), bottoms (+ 343 °C), coke, carbon monoxide, carbon dioxide and water.

The coke yield was calculated from the mass flow rate of the combustion gas and its chromatographic composition, as described above.

Samples of the total liquid effluent were collected to perform simulated distillation (ASTM D2887).

In general, studies in the literature calculate the water produced by the difference between 100% by weight and the sum of the other yields, or simply do not report how the water yield calculation was performed. In this example, the water yield was estimated considering the oxygen present in the load (11.5 wt% in soybean oil and 57.7 wt% in bio-oil) minus the oxygen present in the CO and $CO_2$ products.

3.4 - Experimental result:

The results in Table VIII show the product yields, the gasoline composition and the operating conditions used in the tests.

In the test related to the state of the art (C1), only soybean oil was used as the catalytic cracking feed and a medium-pore zeolite-based catalyst.

In the tests related to this invention (C2 to C5), bio-oil (BO) is co-processed with the grease stream (OS) at levels of 1, 5, 10 and 20% m/m.

An increase in the coke content is clearly observed with the addition of bio-oil. The coke increases 50% when 10% bio-oil is added and 115% when 20% bio-oil is added. Although the increase on an industrial scale is probably different, the bench experiment clearly shows the tendency that the addition of bio-oil helps the heat balance of the UFCC in the processing of grease loads.

Another very clear effect is the increase in bio-oil on the percentage of aromatics in the gasoline, which increases, raising the octane rating.

Table VIII

| Operating conditions in the ACE, yields (% by weight in relation to the load fed) and gasoline composition. | | | | | |
|---|---|---|---|---|---|
| Test | C1 | C2 | C3 | C4 | C4 |
| Temperature | 500 | 500 | 500 | 500 | 500 |
| Grease stream (wt%) | 100 | 98 | 94 | 89 | 79 |
| Bio-oil (wt%) | 0 | 1 | 5 | 10 | 20 |
| Tween 80 (wt%) | | 1 | 1 | 1 | 1 |
| Yields | | | | | |
| Coke (wt%) | 2.0 | 2.2 | 2.8 | 3.0 | 4.3 |
| Fuel gas (wt%) | 5.2 | 5.1 | 5.3 | 5.2 | 5.1 |
| Ethene (wt%) | 4.8 | 4.6 | 4.9 | 4.8 | 4.7 |
| GLP (C3-C4) (wt%) | 30.0 | 28.6 | 28.7 | 27.0 | 24.2 |
| Propene (wt%) | 12.8 | 12.0 | 12.2 | 11.9 | 11.1 |
| Olefins (wt%) | 11.3 | 10.8 | 10.7 | 10.0 | 8.9 |
| Liquid yield (wt%) | 60.9 | 62.2 | 61.1 | 62.6 | 63.8 |
| Naphta (C5-220 °C) (wt%) | 43.8 | 44.8 | 42.2 | 42.1 | 39.8 |
| LCO (220 °C to 344 °C) (wt%) | 3.03 | 3.03 | 2.79 | 2.70 | 2.43 |
| Bottoms (+ 344 °C) (wt%) | 1.76 | 1.74 | 1.95 | 1.51 | 1.43 |
| Water (wt%) | 12.2 | 12.6 | 14.2 | 16.2 | 20.2 |
| CO (wt%) | 1.63 | 1.65 | 1.80 | 1.93 | 2.20 |
| $CO_2$ (wt%) | 0.25 | 0.26 | 0.29 | 0.30 | 0.32 |
| Total (wt%) | 100.0 | 100.0 | 100.0 | 100.0 | 100.0 |
| Gasoline PIANIO | | | | | |
| Paraffines | 2.9 | 2.8 | 2.7 | 2.5 | 2.3 |
| Olefins | 15.0 | 14.3 | 14.0 | 12.9 | 10.6 |
| Naphthenics | 2.9 | 2.9 | 2.9 | 2.9 | 2.6 |
| Aromatics | 76.0 | 76.8 | 77.2 | 78.4 | 81.1 |
| Non-identified | 3.2 | 3.2 | 3.2 | 3.3 | 3.4 |
| MON | 86.4 | 86.7 | 86.9 | 87.2 | 88.3 |
| RON | 103.8 | 104.1 | 104.3 | 104.8 | 105.7 |

Claims

1. PROCESS FOR GENERATING RENEWABLE PRODUCTS FROM BIO-OIL AND GREASE STREAMS IN CATALYTIC CRACKING IN FLUID CATALYTIC CRACKING UNITS, **characterized by** comprising the steps of:

(a) preheating and atomizing a renewable grease stream and preheating and atomizing a bio-oil stream;
(b) placing the atomized streams in contact with a regenerated catalyst, containing zeolite with intermediate pore size in order to initiate the catalytic cracking reactions;
(c) separating the cracked products;

(d) regenerating the catalyst by removing coke and conducting the catalyst back to the base of the riser to restart cracking.

2.  PROCESS, according to claim 1, **characterized in that** the grease stream introduced into the catalytic cracking unit represents between 70% by weight and 98% by weight and the bio-oil, derived from lignocellulosic biomass, represents between 2% by weight and 30% by weight of the total load of the fluid catalytic cracking unit.

3.  PROCESS, according to claim 1, **characterized in that** the bio-oil used in the mixture is obtained via thermochemical conversion processes of lignocellulosic biomass.

4.  PROCESS, according to claim 1, **characterized in that** the bio-oil is added at a point at the same level or downstream of the grease stream.

5.  PROCESS, according to claim 1, **characterized in that** the proportion of bio-oil of lignocellulosic origin introduced into the fluid catalytic cracking unit is in a ratio of 5% to 20% by weight, and more preferably 5% to 10% by weight, in relation to the total processed load.

6.  PROCESS, according to claim 1, **characterized in that** the grease stream is castor oil, soybean oil, cottonseed oil, peanut oil, beef tallow or other oil of plant origin or animal fat, pure or used, or ester obtained by the transesterification of these oils and fats.

7.  PROCESS, according to claim 1, **characterized in that** the zeolite catalyst comprises from 10% to 75% w/w of intermediate pore zeolite, 1% to 40% w/w of alumina, 0% to 20% w/w of silica, 0% to 20% w/w of phosphorus; and kaolin for closing the balance.

8.  PROCESS, according to claim 6, **characterized in that** the intermediate pore zeolite catalyst is selected from the types of structure consisting of: MFI, MEL, ZSM-8, ZSM-12, ZSM-21, ZSM-23, ZSM-35, ZSM-38, IMF and TUN and any of these combinations.

9.  PROCESS, according to claim 1, **characterized in that** the fluid catalytic cracking reactor corresponds to the following operating conditions: pressures between 200 kPa and 400 kPa, catalyst/biomass ratio in the range of 5 to 30, contact time between 1 and 3 seconds.

10. RENEWABLE NAPHTHA, obtained according to the process defined in claim 1, **characterized by** containing a typical gasoline distillation range with an initial boiling point of 30 °C and a final boiling point of 220 °C.

11. RENEWABLE NAPHTHA, according to claim 10, **characterized by** containing between 20% and 90% w/w of compounds with renewable aromatic rings.

12. RENEWABLE NAPHTHA, according to claim 10, **characterized by** containing less than 1% w/w of oxygen.

13. RENEWABLE NAPHTHA, according to claim 10, **characterized by** containing less than 10 ppm of sulfur.

14. RENEWABLE NAPHTHA, according to claim 10, **characterized by** having at least 83 of MON and 92 of RON.

15. RENEWABLE MEDIUM DISTILLATE, obtained according to the process defined in claim 1, **characterized by** being in the typical distillation range of LCO with an initial boiling point of 220 °C and an end point of 344 °C.

16. RENEWABLE MEDIUM DISTILLATE, according to claim 15, **characterized by** containing less than 1% w/w of oxygen.

17. RENEWABLE MEDIUM DISTILLATE, according to claim 15, **characterized by** containing less than 10 ppm of sulfur.

18. RENEWABLE BUNKER, obtained according to the process defined in claim 1, **characterized by** having an initial boiling point of 344 °C.

19. RENEWABLE BUNKER, according to claim 18, **characterized by** containing less than 1% w/w of oxygen.

**20.** RENEWABLE BUNKER, according to claim 18, **<u>characterized</u> by** containing less than 10 ppm of sulfur.

FIGURE 1

**FIGURE 2**

## INTERNATIONAL SEARCH REPORT

| | International application No. |
|---|---|
| | **PCT/BR2023/050153** |

| A. | CLASSIFICATION OF SUBJECT MATTER |
|---|---|

C10G 3/00 (2006.01)i; C10G 11/18 (2006.01)i; C10G 11/05 (2006.01)i; C10L 1/02 (2006.01)i
CPC: C10G 3/57; C10G 11/18; C10G 11/05; C10L 1/02; C10G 2300/1011; C10G 2400/02; C10G 2400/04; C10L
2200/0415; C10L 2200/0438; C10L 2200/0469

According to International Patent Classification (IPC) or to both national classification and IPC

| B. | FIELDS SEARCHED |
|---|---|

Minimum documentation searched (classification system followed by classification symbols)

C10G 3/00; C10G 11/18; C10G 11/05; C10L 1/02
CPC: C10G 3/57; C10G 11/18; C10G 11/05; C10L 1/02

Documentation searched other than minimum documentation to the extent that such documents are included in the fields searched

Base de dados do INPI - BR (SINPI), PORTAL CAPES

Electronic data base consulted during the international search (name of data base and, where practicable, search terms used)

CAPLUS, PCTFULL, ESPACENET, DERWENT

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>A | WO 2013102662 A1 (SHELL INT RESEARCH [NL]) 11 July 2013 (2013-07-11)<br>    The whole document | 1-10<br>11-20 |
| X | US 2019233751 A1 (XYLECO INC [US]) 01 August 2019 (2019-08-01)<br>    The whole document | 10-14 |
| X | BR 102016003995 A2 (PETROLEO BRASILEIRO SA PETROBRAS [BR]) 16 April 2019<br>(2019-04-16)<br>    The whole document | 15-20 |
| A | WO 2020068820 A1 (EXXONMOBIL RES & ENG CO [US]) 02 April 2020 (2020-04-02)<br>    The whole document | 1-9 |
| A | WO 2021007549 A1 (SPRAYING SYSTEMS CO [IL]) 14 January 2021 (2021-01-14)<br>    The whole document | 1-9 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 August 2023** | **16 August 2023** |

| Name and mailing address of the ISA/BR | Authorized officer |
|---|---|
| **National Institute of Industrial Property (Brazil)**<br>**Rua Mayrink Veiga, 9, 6° andar, CEP 20.090-910 Rio de Janeiro – RJ**<br>**Brazil** | **Stella Fernandes Simão** |
| Telephone No. **(55 21) 3037-3742, 3037-3984** | Telephone No. **+55 21 3037 4528 - 3037 3319** |

Form PCT/ISA/210 (second sheet) (July 2022)

**INTERNATIONAL SEARCH REPORT**

| International application No. |
| --- |
| **PCT/BR2023/050153** |

**C.     DOCUMENTS CONSIDERED TO BE RELEVANT**

| Category* | Citation of document, with indication, where appropriate, of the relevant passages | Relevant to claim No. |
| --- | --- | --- |
| A | WO 2021043018 A1 (REZEL CATALYSTS CORP  [CN]) 11 March 2021 (2021-03-11)<br>The whole document | 1-20 |

Form PCT/ISA/210 (second sheet) (July 2022)

**INTERNATIONAL SEARCH REPORT**
Information on patent family members

International application No.

**PCT/BR2023/050153**

| Patent document cited in search report | | | Publication date (day/month/year) | Patent family member(s) | | | Publication date (day/month/year) |
|---|---|---|---|---|---|---|---|
| WO | 2013102662 | A1 | 11 July 2013 | CN | 104093816 | A | 08 October 2014 |
| | | | | EP | 2800797 | A1 | 12 November 2014 |
| | | | | JP | 2015506397 | A | 02 March 2015 |
| | | | | US | 2013178672 | A1 | 11 July 2013 |
| US | 2019233751 | A1 | 01 August 2019 | US | 10597595 | B2 | 24 March 2020 |
| | | | | BR | 112020008273 | A2 | 20 October 2020 |
| | | | | CA | 3080669 | A1 | 02 May 2019 |
| | | | | JP | 2021501231 | A | 14 January 2021 |
| | | | | MX | 2020004287 | A | 29 July 2020 |
| | | | | US | 2021009911 | A1 | 14 January 2021 |
| | | | | WO | 2019084518 | A1 | 02 May 2019 |
| BR | 102016003995 | A2 | 16 April 2019 | BR | 102016003995 | B1 | 13 October 2020 |
| | | | | US | 2017240824 | A1 | 24 August 2017 |
| | | | | US | 10253273 | B2 | 09 April 2019 |
| WO | 2020068820 | A1 | 02 April 2020 | CA | 3112833 | A1 | 02 April 2020 |
| | | | | EP | 3856874 | A1 | 04 August 2021 |
| | | | | US | 2020095510 | A1 | 26 March 2020 |
| WO | 2021007549 | A1 | 14 January 2021 | AU | 2020310205 | A1 | 10 February 2022 |
| | | | | CA | 3147048 | A1 | 14 January 2021 |
| | | | | CN | 114375320 | A | 19 April 2022 |
| | | | | EP | 3997195 | A1 | 18 May 2022 |
| | | | | JP | 2022540219 | A | 14 September 2022 |
| | | | | US | 2021008517 | A1 | 14 January 2021 |
| | | | | US | 11253832 | B2 | 22 February 2022 |
| WO | 2021043018 | A1 | 11 March 2021 | US | 2022306942 | A1 | 29 September 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010023456 A
- US 7540952 B
- BR 1020140223665
- BR 1020160039959
- BR 1120170232529

**Non-patent literature cited in the description**

- **GRAÇA, I** ; **RAMÔA RIBEIRO, F.** ; **CERQUEIRA, H.S.** ; **LAM, Y.L.** ; **DEALMEIDA, M.B.B.** Catalytic cracking of mixtures of model bio-oil compounds and gasoil. *Applied Catalysis B: Environmental.*, 2009, vol. 90 (3-4), 556-563